(19) **Europäisches Patentamt European Patent Office Office européen des brevets**

(11) **EP 1 048 348 B1**

(12) **FASCICULE DE BREVET EUROPEEN**

(45) Date de publication et mention de la délivrance du brevet:
**11.07.2007 Bulletin 2007/28**

(51) Int Cl.:
*B01J 23/62* (2006.01)  *C07C 5/333* (2006.01)
*B01J 27/13* (2006.01)

(21) Numéro de dépôt: **00401072.4**

(22) Date de dépôt: **18.04.2000**

(54) **Catalyseur comprenant un élément des groupes 8, 9 et 10 présentant une bonne accessibilité et son utilisation dans un procédé de déshydrogenation des paraffines**

Katalysator mit guter Ereichbarkeit und mit Elementen der Grupen 8, 9 und 10, und seine Verwendung in einem Verfahren für die Dehydrierung von Paraffinen

Catalyst with good accessibility containing elements of the groups 8, 9 and 10 and the use thereof in a process for the dehydrogenation of paraffins

(84) Etats contractants désignés:
**DE GB NL**

(30) Priorité: **26.04.1999 FR 9905294**

(43) Date de publication de la demande:
**02.11.2000 Bulletin 2000/44**

(73) Titulaire: **Institut Français du Pétrole**
**92852 Rueil-Malmaison Cedex (FR)**

(72) Inventeurs:
• **Uzio, Denis**
 **78160 Marly le Roi (FR)**
• **Didillon, Blaise**
 **92500 Rueil-Malmaison (FR)**
• **Pellier, Emmanuel**
 **78320 La Vaerriére (FR)**

(56) Documents cités:
**EP-A- 0 714 872**   **EP-A- 0 749 779**
**US-A- 3 892 657**   **US-A- 4 551 574**

Remarques:
Le dossier contient des informations techniques présentées postérieurement au dépôt de la demande et ne figurant pas dans le présent fascicule.

EP 1 048 348 B1

## Description

<u>Domaine technique</u>

L'invention concerne les catalyseurs utilisés dans la conversion des hydrocarbures et en particulier dans la réaction de déshydrogénation des paraffines. L'invention porte sur la méthode de préparation mise en oeuvre pour synthétiser un catalyseur. L'invention porte également sur l'utilisation en déshydrogénation des paraffines dudit catalyseur.

Les alcènes constituent une charge de choix pour l'industrie pétrochimique. Les principales sources d'alcènes sont les procédés de vapocraquage et de craquage catalytique. Cependant, ces deux procédés produisent également des sous-produits et la demande croissante est orientée vers des alcènes spécifiques qu'il serait peu économique de produire par craquage.

C'est pourquoi la production directe d'alcènes reste dans certains cas une étape incontournable. C'est le cas du propylène, isobutène ou des alcènes linéaires à chaîne longue pour la production de polypropylène, de MTBE et de LAB (Linear Alkyl Benzene) respectivement.

Les principales spécificités de la réaction de déshydrogénation résident dans le fait que l'équilibre thermodynamique limite le taux de conversion par passe et que la réaction est fortement endothermique. Ces deux caractéristiques sont déterminantes dans les choix technologiques au niveau du procédé mais également dans le "design" et la conception du catalyseur.

Ainsi, la réaction de déshydrogénation des n-paraffines comportant entre 10 et 14 atomes de carbone se réalise généralement à des températures d'environ 450-500°C avec un taux de conversion par passe compris entre 10 % et 25 %, limité par la thermodynamique.

Le fait de travailler à haute température est nécessaire pour maintenir un niveau de conversion proche de l'équilibre thermodynamique, mais ces hautes températures favorisent également un certain nombre de réactions parasites conduisant à un produit de moindre qualité. Parmi ces réactions, il existe des réactions conduisant à la formation de produits légers (craquage, hydrogénolyse), de composés fortement insaturés précurseurs de dépôts carbonés et donc initiateurs de désactivation (déshydrocyclisation, déshydrogénation profonde) comme les composés aromatiques ou les dioléfines et des réactions d'isomérisation squelettale responsables de la formation de molécules ramifiées. Dans ces conditions opératoires particulièrement sévères, il est très difficile de maintenir une activité élevée pendant des durées importantes en raison de ces réactions secondaires.

<u>Art antérieur</u>

Les moyens de limiter ces réactions secondaires peuvent porter sur le procédé et/ou sur la formulation catalytique. Ainsi le brevet EP 0 462 094 B1 revendique l'addition d'hydrogène dans la charge des rapports molaires $H_2$/ hydrocarbures compris entre 0,5 et 1,9. Le rôle de cet appoint d'hydrogène est de limiter ou de retarder la formation de coke à la surface du catalyseur sans qu'un effet négatif sur la conversion des n-paraffines ne soit observé.
Une autre solution proposée par les brevets US 3,448,165, US 3,907,921 et US 5,233,118 consiste à injecter une faible quantité d'eau et/ou de soufre avec la charge hydrocarbonée à déshydrogéner. L'eau peut être injectée à débit constant ou croissant graduellement avec le temps de fonctionnement du catalyseur. Il a été reporté qu'un optimum en terme de performance était obtenu en augmentant l'injection d'eau avec la température du réacteur au cours du cycle de fonctionnement.

L'autre voie explorée, toujours pour améliorer les performances des systèmes catalytiques et notamment leur stabilité, consiste à déterminer les propriétés physico-chimiques optimales. Ainsi, le brevet US 4,716,143 invoque un catalyseur à base de platine supporté tel que la distribution du platine soit limitée à la surface externe du support sur une épaisseur maximale de 400 $\mu$m. L'avantage d'un tel choix réside dans le fait qu'une distribution en périphérie du support permet de limiter les réactions parasites et par conséquent d'améliorer les performances du catalyseur. Cependant, ce type de distribution ne permet que rarement l'obtention des rapports atomiques platine/modificateurs homogènes à l'échelle des particules (nanomètre). De plus, une surconcentration de phase active en surface peut engendrer des limitations diffusionnelles au niveau du grain de catalyseur (diffusion extragranulaire) et réduire de ce fait le rendement global de la réaction.

Parmi les modificateurs du platine les plus couramment utilisés, on trouve les éléments du groupe 14 et 13 et en particulier l'étain (US 3,745,112). Le rôle de l'étain présent à la surface du catalyseur à l'état d'oxydation + 2 ou plus préférentiellement + 4 est de minimiser les réactions d'isomérisation et de craquage qui se produisent au niveau des sites acides du support. Un autre exemple de modificateur du platine est l'indium, cité notamment dans les brevets US 4,551,574, EP B 183 861 et JP-B-91041211. En effet, ce dernier confère une meilleure stabilité en inhibant également les réactions secondaires de déshydrogénation profonde (polyoléfines) et d'isomérisation squelettale (hydrocarbures ramifiés). Notons que le pouvoir promoteur de ces éléments vis-à-vis du platine est bien connu également dans le cadre très étudié des catalyseurs de reformage catalytique.

Pour les catalyseurs à base de platine, on a intérêt, compte tenu du coût élevé du platine, à disperser au mieux la phase métallique, c'est-à-dire à augmenter la proportion de métal noble en contact avec la surface et les molécules à transformer. Il est en effet important de développer la surface métallique spécifique (surface exprimée par gramme de métal) maximale afin d'obtenir le taux de conversion le plus élevé possible. Ainsi, une chute de l'accessibilité, équivalent à une augmentation de la taille des particules ou un regroupement des particules élémentaires, est fortement préjudiciable à la productivité de la réaction. On cherchera donc à minimiser la taille des particules au cours de la préparation et à maintenir cet état de dispersion élevé, mais thermodynamiquement instable. La présence de chlore en quantité suffisante peut constituer une réponse à ce problème. En effet, le chlore est connu pour avoir un effet stabilisant et même redispersant envers les particules de platine de très petite taille. Le brevet US 4,430,517 cite en exemple un taux de chlore supérieur à 2.5 % pds pour une teneur en platine de 0,75% poids, correspondant à un rapport atomique Cl/Pt d'environ 19. Cet effet stabilisant est également connu depuis longtemps pour les catalyseurs de reformage catalytique (US 2,479,109, US 2,602,772). Cependant, si dans ce dernier cas les réactions de cyclisation, d'isomérisation squelettale sont fortement recherchées, dans le cas de la déshydrogénation en général et plus encore dans le cas de la déshydrogénation des paraffines ayant un nombre d'atomes de carbone entre 6 et 22, ces réactions constituent des réactions parasites qu'il convient de limiter au risque de subir une désactivation rapide du catalyseur. L'ajout de chlore pose donc un problème au vu des réactions secondaires qu'il favorise.

Afin de limiter les réactions secondaires, il est intéressant de déposer un alcalin où alcalino-terreux dont le rôle consistera à participer à la neutralisation des sites acides du support de force faible et moyenne. Ainsi, une addition même limitée de lithium (0,1 % en poids) permet de neutraliser ces sites acides qui sont responsables de la formation de produits isomérisés et légers (réactions de craquage). La formation d'aromatiques est également diminuée par l'ajout de lithium. Cependant, il est également connu que cette addition entraîne une diminution de l'activité totale du catalyseur. Cette diminution est souvent reliée à un phénomène de recouvrement de la phase métallique par le métal alcalin.

Les méthodes de préparation classiquement utilisées ne permettent pas de déposer des teneurs suffisantes en alcalin, et notamment en lithium, sans qu'il se produise une chute importante de l'accessibilité du platine en présence de faible quantité d'halogène. Des travaux de la littérature font état de ce phénomène lors de l'imprégnation de lithium sur des catalyseurs à base de platine (Passos, Schmal, Fréty, Catalysis Letters 14 (1994) 57-64).

Pour pallier ce phénomène, une voie proposée par le brevet US 5,536,695 consiste à déposer du lithium sur un support alumine et de procéder à un traitement thermique à haute température afin de former une phase aluminate de surface ($LiAl_5O_8$ ou $LiAlO_2$). Le dépôt de platine peut alors être effectué à l'issue de cette étape en utilisant un précurseur préférentiellement organique. L'utilisation d'une solution acide d'un précurseur minéral (acide hexachloroplatinique, ou hexahydroxylatinique) présente l'inconvénient de solubiliser partiellement le support aluminate formé et donc de conduire à une perte en métal alcalin. D'autre part, l'inversion des étapes de formation de l'aluminate et de dépôt du platine ne peut être envisagée, étant donné que le traitement thermique nécessaire à la formation de la phase aluminate, mené à 800°C environ, entraînerait un phénomène très important de frittage du platine par agrégation des particules. De plus, l'utilisation de solutions organiques est d'un faible intérêt industriel en regard des problèmes environnementaux et de sécurité qui y sont rattachés.

Enfin, le support peut également jouer un grand rôle sur la stabilité du système catalytique comme il est enseigné dans les brevets US 4,672,146 et US 358,920. Les paramètres taille de pore et fraction de vide développé par des pores de taille supérieure à 60 ou 100 nm (macropores) sont déterminants sur les propriétés finales de transport surtout dans le cas de molécules hydrocarbonées stériquement encombrées (chaînes longues par exemple). EP-A-0 714 872 et EP-A-0 749 779 décrivent des catalyseurs de déshydrogénation

Résumé de invention

L'invention concerne le procédé de préparation d'un catalyseur comprenant un support, au moins un élément des groupes 8, 9 et 10 de la classification périodique des éléments, ("Handbook of Physics and Chemistry"), 76ème édition, au moins un élément du groupe 14 de la classification périodique des éléments, au moins un élément du groupe 13 de la classification périodique des éléments, au moins un métal alcalin ou alcalino-terreux de la classification périodique des éléments tel que décrit dans le libellé de la revendication indépendante 1. Les revendications 2 à 11 contiennent des caractéristiques optionelles du procédé revendiqué. Au moins un halogène peut être contenu à une teneur comprise entre 0 et 0,2 % poids par rapport au poids total de catalyseur, ledit catalyseur étant caractérisé en ce que l'accessibilité de l'élément des groupes 8, 9 et 10 est supérieure à 50 %. L'invention concerne également l'utilisation telle que définie dans le libellé de la revendication indépendante 12. L'utilisation du catalyseur dans un procédé de déshydrogénation de paraffines ayant de 3 à 22 atomes de carbone par molécule est en particulier revendiquée. On entend par accessibilité au sens de la présente invention la quantité d'élément des groupes 8, 9 et 10 accessible à la charge à convertir par rapport à la quantité totale d'élément des groupes 8, 9 et 10 présente sur le catalyseur.

**EP 1 048 348 B1**

Intérêt

L'invention permet préparer un nouveau catalyseur supporté contenant à la fois un métal alcalin ou alcalino-terreux à une teneur supérieure à 500 ppm, des particules de métal des groupes 8, 9 et 10 d'accessibilité supérieure à 50 % tout en maintenant un taux de chlore inférieur à 0,2 % poids. En effet, il a été découvert que le fait de contenir peu de chlore, agent stabilisateur des petites particules de platine, ne se traduisait pas, pour le type de support considéré, par un vieillissement accéléré du catalyseur par frittage mais permettait en outre de diminuer fortement les réactions secondaires précitées. Enfin, il a également été trouvé que ce catalyseur pouvait subir un traitement régénératif, par combustion du coke, sans altération des performances catalytiques.

Description

Le catalyseur préparé selon le procédé de l'invention comprend un support, au moins un élément des groupes 8, 9 et 10, au moins un élément additionnel choisi parmi les éléments du groupe 14, au moins un élément additionnel choisi parmi les éléments du groupe 13, au moins un alcalin ou alcalino-terreux et éventuellement au moins un halogène à une teneur maximale de 0,2 % en poids par rapport au poids total de catalyseur.

Le métal des groupes 8, 9 et 10 est choisi parmi le platine, le palladium, le rhodium, le ruthénium, l'osmium, l'iridium, le fer, le cobalt, et le nickel, de préférence choisi parmi les métaux nobles des groupes 8, 9 et 10 et préférentiellement le platine, dont la teneur est comprise entre 0,01 et 5 % en poids par rapport au poids total de catalyseur, préférentiellement entre 0,05 et 1 %.

Le métal du groupe 14 est choisi parmi l'étain, le germanium et le plomb, de préférence l'étain à une teneur comprise entre 0,01 et 5 % poids par rapport au poids total de catalyseur, et plus préférentiellement entre 0,1 et 1 %.

Le métal du groupe 13 est choisi parmi l'indium, le gallium et le thallium, de préférence l'indium à une teneur comprise entre 0,005 et 3 % poids par rapport au poids total de catalyseur, et plus préférentiellement entre 0.01 et 1%.

Le métal alcalin est choisi parmi le lithium, le sodium, le potassium, et le césium, de préférence le potassium, à une teneur comprise entre 0,05 et 3 % poids par rapport au poids total de catalyseur, et plus préférentiellement entre 0,1 et 1 %. L'halogène, si le catalyseur en contient est de préférence le chlore à une teneur comprise entre 0 et 0,2 % poids et de préférence entre 0 et 0,15 %.

L'invention est caractérisée en ce que l'accessibilité du métal du groupe VIII est supérieure à 50%, de préférence supérieure à 60% et de manière encore plus préférée supérieure à 70%. La mesure de l'accessibilité du métal du groupe VIII est effectuée par titrage $H_2 O_2$.

Les supports de catalyseur selon l'invention sont généralement des solides poreux choisis parmi les oxydes réfractaires, tels que les alumines, les silices, la magnésie ainsi que l'oxyde de titane et l'oxyde de zinc. Ces deux derniers oxydes peuvent être utilisés seuls ou en mélange avec de l'alumine. De plus, les supports sont préférentiellement des alumines de transition ou des silices dont la surface spécifique est comprise entre 25 et 300 $m^2/g$, de préférence entre 80 et 200 $m^2/g$. Les composés naturels tels que le kieselguhr ou le kaolin peuvent également convenir comme supports de catalyseurs selon l'invention.

La préparation du catalyseur selon l'invention comprend des étapes successives de dépôt selon technique connue de l'homme du métier, d'un métal du groupe 13, puis d'une étape de dépôt d'un métal du groupe 14 et d'une étape de dépôt d'un métal des groupes 8, 9 et 10. Ces étapes de dépôt peuvent être effectuées dans n'importe quel ordre. Le dépôt des métaux est effectué de telle manière que l'accessibilité du métal des groupes 8, 9 et 10 soit supérieure à 50 %. Le dépôt des métaux peut être réalisé par imprégnation à sec ou par excès ou par la méthode d'échange ionique. Une calcination est effectuée par balayage sous air à une température d'environ 500°C dont le but est de décomposer les précurseurs et de former les phases métalliques, ainsi que les interactions souhaitées.

Selon l'invention, on procède au dépôt du métal alcalin ou alcalino-terreux par contact du support avec au moins une solution contenant au moins un précurseur d'au moins un métal alcalin ou alcalino-terreux. Le dépôt du métal alcalin ou alcalino-terreux est caractérisé en ce que le pH de la solution comprenant le précurseur de métal alcalin ou alcalino-terreux est inférieur à 2. La solution comprenant le métal alcalin peut contenir également au moins un métal des groupes 8, 9 et 10 et/ou au moins un métal du groupe 14, et/ou au moins un métal du groupe 13. Le pH de la solution comprenant le métal alcalin peut être amené à une valeur inférieure ou égale à 2 par toute méthode connue de l'homme du métier. Par exemple, on pourra procéder à un ajout d'acide choisi parmi l'acide nitrique, chlorhydrique, sulfurique ou fluorhydrique. De préférence, on ajustera la valeur du pH par ajout d'acide nitrique.

Un traitement thermique final aura pour objet de décomposer le précurseur de métal alcalin et d'éliminer la majeure partie d'halogène injecté pendant les opérations précédentes. Cette étape sera effectuée par exemple en soumettant le catalyseur à un mélange air/eau, 1/1 molaire, à une température d'environ 500°C pendant une durée de 2 à 4 heures. Cette opération de déhalogénation pourra être éventuellement effectuée à l'issue des dépôts des métaux des groupes 14 et 13 et des groupes 8, 9 et 10. Dans ce dernier cas, un traitement thermique sous air sec du même type que précédemment achèvera la préparation du catalyseur.

Le métal des groupes 8, 9 et 10 peut être introduit par tout précurseur connu de l'homme du métier, comme par exemple les sels halogénés, les composés organométalliques. De préférence on utilisera un précurseur du métal des groupes 8, 9 et 10 soluble en milieu aqueux tel l'acide chloroplatinique.

Le métal alcalin, le métal du groupe 14, le métal du groupe 13 peuvent être introduits à l'aide de tous précurseurs connus. Par exemple on utilisera les sels décomposables sous forme de chlorures, bromures, nitrates, de carbonates ou d'acétates. On peut également utiliser les composés organométalliques des métaux des groupes 14 et 13 tels que le tétrabutylétain ou le triphénylindium.

Dans une mise en oeuvre particulière de l'invention, on utilise comme précurseur du métal du groupe 14 et/ou comme précurseur du métal du groupe 13 un composé organométallique ayant une liaison carbone métal du groupe 14 ou 13, soluble en milieu aqueux. Par exemple, on peut utiliser un complexe de l'étain appartenant à la famille des alkyl allyes.

L'invention concerne également l'utilisation du catalyseur dans un procédé de conversion d'hydrocarbures. Par exemple, le catalyseur peut être mis en oeuvre pour une réaction de déshydrogénation de charges paraffiniques contenant 3 à 22 atomes de carbone, de préférence de 5 à 20 atomes de carbone par molécule. Cette réaction s'effectue généralement à une pression opératoire comprise entre 0,02 et 2 MPa, de préférence entre 0,05 et 0,5 MPa et à une température comprise entre 300 et 800°C, de préférence entre 400 et 550°C. Le rapport molaire hydrogène/hydrocarbures est généralement compris entre 0 et 20 et de préférence entre 0 et 10. La vitesse spatiale horaire (exprimée en litre d'hydrocarbure par litre de catalyseur et par heure) est généralement comprise entre 10 et 100 $h^{-1}$. L'ajustement des conditions opératoires est très variable avec la nature des charges traitées et les performances catalytiques visées par l'utilisateur notamment en terme de rendement en oléfines.

## Exemple 1 (selon l'invention)

On prépare un catalyseur A de déshydrogénation contenant 0,30 % poids de platine, 0.33% poids d'étain, 0.11 % poids d'indium, 0,35 % poids de lithium et 0,08 % poids de chlore, déposés sur une alumine delta dont la surface spécifique est de 130 $m^2$/g.

Le support (200 g) est mis en contact avec 900 ml d'eau distillée en maintenant la température du lit constante. 0,8 g d'étain sous forme $SnCl_2$ sont progressivement ajouté en présence d'HCl. Après 4 h de mise en contact le catalyseur est ensuite séché. On met en contact à nouveau le catalyseur avec 650 ml d'eau distillée auxquels sont rajoutés 50 ml d'une solution aqueuse contenant 0,51 g d'indium sous forme $In(NO_3)_3$. Après 3 h de mise en contact, le catalyseur est séché. Enfin, le dépôt de platine se fait par une nouvelle mise en contact du catalyseur avec 650 ml d'eau distillée auxquels on a rajouté 0,6 g de Pt sous forme $H_2PtCl_6$. Après 3 h d'imprégnation, le catalyseur est filtré, séché à 150°C pendant 2 h sous un débit de 200 I/h d'air avec une montée en température de 5°C/mn. Après ce palier, le catalyseur est calciné à 530°C sous un débit de 200 I/h d'air avec une montée en température de 5°C/mn pendant 2 h.

L'imprégnation finale du lithium se fait en ajoutant au catalyseur 0,7 g de lithium sous forme $LiNO_3$ dissous dans 205 ml d'eau distillée dont le pH aura été ajusté à 1,3 par ajout d'acide nitrique à 66 % poids. Le catalyseur est séché à 150°C pendant 2 h sous un débit de 200 I/h d'air avec une montée en température de 5°C/mn puis calciné à 550°C sous un mélange air/eau (50/50 molaire) à une VVH de 100 $h^{-1}$ et avec une montée en température de 5°C/mn pendant 4h. Après arrêt de l'injection d'eau, le catalyseur est calciné à 550°C, pendant 1 h. Tous les éléments sont répartis de manière homogène dans les billes de support.

L'accessibilité du platine mesuré par titrage $H_2/O_2$, selon la méthode décrite ci-après est de 70%.

Le titrage $H_2/O_2$ consiste à mesurer le volume d'oxygène consommé par la réaction (1) après une étape de réduction du catalyseur.

$$Pt_s\text{-}H + \tfrac{3}{4}\, O_2 \rightarrow Pt_s\text{-}O + \tfrac{1}{2}\, H_2O \qquad (1)$$

où $Pt_s$ désigne les atomes superficiels de platine.

Le catalyseur est donc réduit sous hydrogène à 450°C puis, après une redescente sous hydrogène à température ambiante, des volumes connus d'oxygène sont injectés. La consommation d'oxygène est suivie par chromatographie, l'intégration des signaux permettant par différence avec le volume total injecté de connaître le volume d'oxygène consommé par la réaction (1). A l'aide des coefficients stoechiométriques de la réaction (1), on peut alors déterminer la fraction de platine en surface ou accessibilité par la relation :

$$D = \frac{4\,VO_2 M_{Pt}}{3\,V_M[\%Pt\,/\,100]}$$

avec:

M$_{Pt}$: masse molaire du platine (195.09 g.mol$^{-1}$)
V$_M$: volume molaire du gaz (24400 ml/mol) à 25°C
VO$_2$: volume mesuré correspondant à la consommation d'oxygène
% Pt: teneur pondérale en platine du catalyseur

**Exemple 2 (selon l'invention)**

On prépare un catalyseur B de déshydrogénation contenant 0,30 % poids de platine, 0,33 % poids d'étain, 0,11 % poids d'indium, 0,60 % poids de lithium et 0,09 % poids de chlore. Les éléments In, Sn et Pt sont déposés selon le même protocole que dans l'exemple 1. Le lithium est déposé en mettant en contact une solution aqueuse de nitrate de lithium contenant 1,2 g de Li sous forme LiNO$_3$ à pH = 1.3.
Tous les éléments sont répartis de manière homogène dans les billes de support.
L'accessibilité du platine mesurée par titrage H$_2$/O$_2$ est de 60%.

**Exemple 3 (selon l'invention)**

On prépare un catalyseur C contenant 0,31 % poids de platine, 0,32 % poids d'étain, 0,13 % poids d'indium, 0,35 % poids de lithium et 0,09 % poids de chlore. Le catalyseur est préparé dans les conditions de l'exemple 1, mis à part l'introduction de l'étain qui est réalisée dans les conditions suivantes : 120 ml d'eau à pH 4 contenant du chlorure de triméthyl étain est mise en contact avec le support. Après séchage et calcination à 450°C, les autres éléments sont introduits selon les méthodes décrites dans l'exemple 1.
Tous les éléments sont répartis de manière homogène dans les billes de support.
L'accessibilité du platine mesurée par titrage H$_2$/O$_2$ est de 73%.

**Exemple 4 (selon l'invention :catalyseur B régénéré)**

Le catalyseur B de l'exemple 2 a subi une régénération, devenant le catalyseur D, comprenant une traitement thermique sous un débit d'air/azote de concentration variable à haute température à 350°C (montée à 5°C/mn) avec un contrôle de la quantité de chaleur dégagée (T consigne - T catalyseur < 2°C). Après ce traitement, l'accessibilité déterminée par titrage H$_2$/O$_2$ est de 55 %.

**Exemple 5 (comparatif)**

On prépare un catalyseur E de déshydrogénation contenant 0,31 % poids de platine, 0,34 % poids d'étain, 0,14 % poids d'indium, 0,65 % poids de lithium et 0,12 % poids de chlore. Les éléments In, Sn et Pt sont déposés selon le même protocole que dans l'exemple 1. Le lithium est déposé en mettant en contact une solution aqueuse de nitrate de lithium sans réacidification avec HNO$_3$.
Tous les éléments sont répartis de manière homogène dans les billes de support.
L'accessibilité du platine mesurée par titrage H$_2$/O$_2$ est de 20%.

**Exemple 6 (comparatif)**

On prépare un catalyseur F de déshydrogénation contenant 0,31 % poids de platine, 0,35 % poids d'étain, 0,14 % poids d'indium, 0,65 % poids de lithium et 0,57 % poids de chlore. La préparation reprend le protocole présenté dans l'exemple 1. Seul le précurseur (et la quantité engagée) de lithium est modifié, s'agissant d'un chlorure au lieu d'un nitrate de lithium. L'accessibilité des particules de platine est de 63 %.

**Exemple 7 (comparatif)**

On prépare un catalyseur G de déshydrogénation contenant 0,31 % poids de platine, 0,36 % poids d'étain, 0,6 % poids de lithium, et 0,15 % poids d'Indium et 0,12 % poids de chlore, selon les techniques connues de l'homme de l'art avec formation d'un aluminate de lithium.
A 150 g de support alumine, 156 ml d'une solution aqueuse d'acétate de lithium contenant 0,9 g de lithium est ajouté. Le système est laissé en contact avec l'alumine pendant 3 heures, puis séché 1 heure à 120°C et calciné 2 heures à 750°C à une VVH (volume d'air par kilogramme de catalyseur et par heure) de 2000h$^{-1}$. L'étain, l'indium et le platine sont déposés par des étapes successives d'imprégnation à sec (mise en contact de l'alumine avec un volume de solution correspondant à son volume poreux) avec des traitements thermiques intermédiaires de séchage de 1 heure à 120°C et un traitement thermique final de calcination de 2 heures à 530°C. L'analyse par diffraction des rayons X permet de mettre en évidence la présence d'aluminate de lithium à $d=2.38_E$-10 m, $d=1.4_E$-10 m, $d=1.98_E$-10 m et $d=2.8_E$-10 m en présence d'alumine non modifiée. L'accessibilité du catalyseur G déterminée par titrage $H_2/O_2$ est de 42%.

### Exemple 8 (comparaison des performances catalytiques)

Les catalyseurs A à G sont soumis à un test de déshydrogénation d'une charge de n-paraffines dont le nombre de carbones est compris entre 10 et 14. La réaction se déroule de manière isotherme à 450°C, P = 2,4 bar abs, le débit de charge hydrocarbure est de 200 ml/h et celui d'hydrogène de 127 l/h, correspondant à un rapport molaire $H_2$/hydrocarbure de 6 et une VVH de 20 $h^{-1}$. Un apport de 2000 ppm d'eau est maintenu tout au long du test. La masse de catalyseur utilisé est de 4,5 g. Le catalyseur est préalablement calciné sous un débit d'air de 15 l/h pendant 2h à 500°C avec une montée en température de 50°C/h. Après une redescente à température ambiante et une purge sous azote, le catalyseur est soumis à un flux d'hydrogène pur (10 l/h) à 430°C pendant 4 h avec une montée en température de 50°C/h. Enfin, la température est amenée à 450°C avec injection de la charge paraffinique et de l'eau.

Les produits de la réaction sont analysés par chromatographie en phase gaz (colonne PONA de 50 m, diamètre interne 0,2 mm, phase OV1) suivant la procédure suivante: montée de 40°C à 200°C (2°/mn), puis montée à 280°C (10°/mn), palier d'une minute à 280°C. Le débit de fuite est de 200 ml/mn et le volume de la boucle d'injection est de 0,5 μl. Les températures du détecteur à ionisation de flamme (FID), ainsi que de l'injecteur sont de 280°C.

Le tableau suivant rassemble l'ensemble des performances pour les catalyseurs présentés.

| | A | B | C | D | E | F | G |
|---|---|---|---|---|---|---|---|
| Conversion des n-paraffines C10-C14* (%) | 8,2 | 7,5 | 8,2 | 7,6 | 2,1 | 7,8 | 5,6 |
| Rendement en oléfines C10-C14(%) | 7,7 | 7,0 | 7,8 | 7,3 | 2 | 6,9 | 5,4 |
| Rendement en aromatiques C10-C14(%) | 0.22 | 0,12 | 0,21 | 0,18 | 0,05 | 0,35 | 0,09 |
| Rendement en dioléfines C10-C14 (%) | 0,13 | 0,22 | 0,13 | 0,08 | 0,02 | 0,24 | 0,07 |
| Rendement en iso paraffines C10-C14 (%) | 0,10 | 0,09 | 0,10 | 0,04 | 0,03 | 0,16 | 0,04 |
| Rendement autres produits C10⁻ et C14⁺ (%) | 0,05 | 0,07 | 0 | 0 | 0 | 0,15 | 0 |
| Pente de désactivation ** (%/h) | 0,0052 | 0,0033 | 0,0051 | 0,0028 | 0,0025 | 0,0045 | ,0015 |
| Accessibilité *** (%) | 70 | 60 | 73 | 55 | 20 | 63 | 42 |

*: déterminé après 100 h de fonctionnement.
**: définie par la pente de la courbe conversion en n-paraffines C10-C14 en fonction du temps
***: déterminée pas titrage $H_2/O_2$

D'après les résultats du tableau ci-dessus, on constate que les catalyseurs selon l'invention démontrent une activité au moins aussi bonne que les catalyseurs selon l'art antérieur et surtout que la sélectivité en produits désirés (oléfines) augmente de manière surprenante pour les catalyseurs selon la présente invention grâce à une diminution des pertes par aromatisation et déshydrogénation en dioléfines (catalyseurs A, B, C, D en comparaison au catalyseur F).

### Revendications

1. Procédé de préparation d'un catalyseur comprenant au moins un support, au moins un élément des groupes 8, 9 et 10 de la classification périodique des éléments, au moins un élément du groupe 14 de la classification périodique des éléments, au moins un élément du groupe 13 de la classification périodique des éléments, au moins un métal alcalin ou alcalino-terreux, ledit procédé comprenant au moins le dépôt par étapes successives d'un métal du groupe 13, d'un métal du groupe 14 et d'un métal des groupes 8, 9 et 10, la calcination par balayage sous air à une température d'environ 500°C et le dépôt du métal alcalin ou alcalino-terreux par contact du support avec au moins une solution contenant au moins un précurseur d'au moins un métal alcalin ou alcalino-terreux et ayant un pH inférieur à 2.

2. Procédé selon la revendication 1 tel que le pH de la solution contenant au moins un précurseur d'au moins un métal alcalin ou alcalino-terreux est amené à une valeur inférieure à 2 par ajout d'un acide choisi parmi l'acide nitrique, l'acide chlorhydrique, l'acide sulfurique et l'acide fluorhydrique.

3. Procédé selon ta revendication 1 ou 2 tel que l'on effectue une étape de déhalogénation dudit catalyseur.

4. Procédé selon l'une des revendications 1 à 3 tel que ledit catalyseur comprend, en poids par rapport au poids total de catalyseur, de 0,01 à 5 % d'au moins un élément des groupes 8, 9 et 10, de 0,01 à 5 % d'au moins un élément du groupe 14, de 0,005 à 3 % d'au moins un élément du groupe 13 et de 0,05 à 3 % poids d'au moins un métal alcalin ou alcalino-terreux.

5. Procédé selon l'une des revendications 1 à 4 tel que ledit catalyseur comprend moins de 0,15 % poids d'halogène par rapport au poids total de catalyseur.

6. Procédé selon l'une des revendications 1 à 5 tel que ledit catalyseur comprend au moins 0,1 % poids de métal alcalin ou alcalino-terreux par rapport au poids total de catalyseur.

7. Procédé selon l'une des revendications 1 à 6 tel que l'élément des groupes 8, 9 et 10 est le platine.

8. Procédé selon l'une des revendications 1 à 7 tel que l'élément du groupe 14 est l'étain.

9. Procédé selon l'une des revendications 1 à 8 tel que l'élément du groupe 13 est l'indium.

10. Procédé selon l'une des revendications 1 à 9 tel que le support dudit catalyseur est un oxyde réfractaire de surface spécifique comprise entre 25 et 300 m$^2$/g.

11. Procédé selon l'une des revendications 1 à 10 tel que le support oxyde dudit catalyseur est l'alumine.

12. Utilisation d'un catalyseur préparé selon le procédé selon l'une des revendications 1 à 11 dans un procédé de conversion d'hydrocarbures.

13. Utilisation selon la revendication 12 dans un procédé de déshydrogénation des paraffines ayant de 3 à 22 atomes de carbone par molécule.

14. Utilisation selon la revendication 13 dans un procédé de déshydrogénation des paraffines ayant de 5 à 20 atomes de carbone par molécule.


**Claims**

1. A process for the preparation of a catalyst comprising at least one support, at least one element from groups 8, 9 or 10 of the periodic table, at least one element from group 14 of the periodic table, at least one element from group 13 of the periodic table, at least one alkali or alkaline-earth metal, said process comprising at least the deposition by successive steps of a metal from group 13, of a metal from group 14 and of a metal from groups 8, 9 and 10, the calcination by passing a stream of air at a temperature of about 500°C and the deposition of an alkali or alkaline-earth metal by bringing the support into contact with at least one solution containing at least one precursor of at least one alkali or alkaline-earth metal and having a pH less than 2.

2. A process according to claim 1, in which pH of the solution containing at least one precursor of at least one alkali or alkaline-earth metal is brought to a value less than 2 by adding an acid selected from nitric acid, hydrochloric acid, sulphuric acid and hydrofluoric acid.

3. A process according to claim 1 or claim 2, in which a dehalogenation step of said catalyst is carried out.

4. A process according to any one of claims 1 to 3 in which said catalyst comprises, by weight with respect to the total catalyst weight, 0.01% to 5% of at least one element from groups 8, 9 or 10, 0.01% to 5% of at least one element from group 14, 0.005% to 3% of at least one element from group 13 and 0.05% to 3% by weight of at least one alkali or alkaline-earth metal.

5. A process according to any one of claims 1 to 4, in which said catalyst comprises less than 0.15% by weight of halogen with respect to the total catalyst weight.

6. A process according to any one of claims 1 to 5, in which said catalyst comprises at least 0.1% by weight of alkali or lkaline-earth metal with respect to the total catalyst weight.

7. A process according to any one of claims 1 to 6, in which the element from groups 8, 9 or 10 is platinum.

8. A process according to any one of claims 1 to 7, in which the element from group 14 is tin.

9. A process according to any one of claims 1 to 8, in which the element from group 13 is indium.

10. A process according to any one of claims 1 to 9, in which the support of said catalyst is a refractory oxide with a specific surface area in the range 25 to 300 $m^2$/g.

11. A process according to any one of claims 1 to 10, in which the oxide support of said catalyst is alumina.

12. Use of a catalyst prepared according to any one of claims 1 to 11 in a hydrocarbon conversion process.

13. Use according to claim 12 in a process for dehydrogenating paraffins containing 3 to 22 carbon atoms per molecule.

14. Use according to claim 13 in a process for dehydrogenating paraffins containing 5 to 20 carbon atoms per molecule.

**Patentansprüche**

1. Verfahren zur Herstellung eines Katalysators, das mindestens einen Träger, mindestens ein Element der Gruppen 8, 9 und 10 des Periodensystems der Elemente, mindestens ein Element der Gruppe 14 des Periodensystems der Elemente, mindestens ein Element der Gruppe 13 des Periodensystems der Elemente, mindestens ein alkalisches oder erdalkalisches Metall umfasst, wobei das Verfahren mindestens die Ablagerung in aufeinander folgenden Schritten eines Metalls der Gruppe 13, eines Metalls der Gruppe 14 und eines Metalls der Gruppen 8, 9 und 10, die Kalzinierung durch Spülen unter Luft bei einer Temperatur von etwa 500 °C und die Ablagerung des alkalischen oder erdalkalischen Metalls durch Kontakt des Trägers mit mindestens einer Lösung umfasst, die mindestens einen Vorläufer mindestens eines alkalischen oder erdalkalischen Metalls enthält, und die einen pH-Wert aufweist, der niedriger als 2 ist.

2. Verfahren nach Anspruch 1, bei dem der pH-Wert der Lösung, die mindestens einen Vorläufer mindestens eines alkalischen oder erdalkalischen Metalls enthält, durch Zugabe einer Säure, ausgewählt aus Salpetersäure, Salzsäure, Schwefelsäure und Fluorwasserstoffsäure auf einen Wert gebracht wird, der niedriger als 2 ist.

3. Verfahren nach einem der Ansprüche 1 oder 2, bei dem ein Dehalogenierungsschritt des Katalysators durchgeführt wird.

4. Verfahren nach einem der Ansprüche 1 bis 3, bei dem der Katalysator bezogen auf das Gesamtgewicht des Katalysators zwischen 0,01 und 5 Gew.-% mindestens eines Elements der Gruppen 8, 9 und 10, zwischen 0,01 und 5 Gew.-% mindestens eines Elements der Gruppe 14, zwischen 0,005 und 3 Gew.-% mindestens eines Elements der Gruppe 13 und zwischen 0,05 und 3 Gew.-% mindestens eines alkalischen oder erdalkalischen Metall umfasst.

5. Verfahren nach einem der Ansprüche 1 bis 4, bei dem der Katalysator mindestens 0,15 Gew.-% Halogen bezogen auf das Gesamtgewicht des Katalysators umfasst.

6. Verfahren nach einem der Ansprüche 1 bis 5, bei dem der Katalysator mindestens 0,1 Gew.-% alkalisches oder erdalkalisches Metall bezogen auf das Gesamtgewicht des Katalysators umfasst.

7. Verfahren nach einem der Ansprüche 1 à 6, bei dem das Element der Gruppen 8, 9 und 10 Platin ist.

8. Verfahren nach einem der Ansprüche 1 à 7, bei dem das Element der Gruppe 14 Zinn ist.

**9.** Verfahren nach einem der Ansprüche 1 bis 8, bei dem das Element der Gruppe 13 Indium ist.

**10.** Verfahren nach einem der Ansprüche 1 bis 9, bei dem der Träger ein temperaturbeständiges Oxid mit einer spezifischen Oberfläche im Bereich zwischen 25 und 300 m$^2$/g ist.

**11.** Verfahren nach einem der Ansprüche 1 bis 10, bei dem das Trägeroxid des Katalysators Aluminiumoxid ist.

**12.** Verwendung eines Katalysators, der gemäß dem Verfahren nach einem der Ansprüche 1 bis 11 in einem Verfahren zur Umwandlung von Kohlenwasserstoffen hergestellt wurde.

**13.** Verwendung nach Anspruch 12 in einem Verfahren zur Dehydrierung von Paraffinen, die 3 bis 22 Kohlenwasserstoffatome je Molekül aufweisen.

**14.** Verwendung nach Anspruch 13 in einem Verfahren zur Dehydrierung von Paraffinen, die 5 bis 20 Kohlenwasserstoffatome je Molekül aufweisen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0462094 B1
- US 3448165 A
- US 3907921 A
- US 5233118 A
- US 4716143 A
- US 3745112 A
- US 4551574 A
- EP 183861 B
- JP 91041211 B
- US 4430517 A
- US 2479109 A
- US 2602772 A
- US 5536695 A
- US 4672146 A
- US 358920 A
- EP 0714872 A
- EP 0749779 A

**Littérature non-brevet citée dans la description**

- **PASSOS ; SCHMAL ; FRÉTY.** *Catalysis Letters,* 1994, vol. 14, 57-64